# EUROPEAN PATENT SPECIFICATION

(11) **EP 2 470 196 B1**
(45) Date of publication and mention of the grant of the patent: **22.01.2020**
(21) Application number: 10754613.7
(22) Date of filing: 26.08.2010
(51) Int. Cl.: A61K 38/20, A61K 38/48, A61K 38/36, A61P 19/00

(54) **OSTEOLYSIS TREATMENT**
OSTEOLYSEBEHANDLUNG
TRAITEMENT DE L'OSTÉOLYSE

(30) Priority: 27.08.2009 US 549116
(43) Date of publication of application: 04.07.2012
(73) Proprietor: Biomet Biologics, LLC, Warsaw, Indiana 46582 (US)
(72) Inventor: HOEPPNER, Jacy, Warsaw Indiana 46580 (US)
(74) Representative: Manitz Finsterwald Patent- und Rechtsanwaltspartnerschaft mbB
(86) International application number: PCT/US2010/046826
(87) International publication number: WO 2011/031525

(56) References cited:
- US-A- 6 096 728
- US-A1- 2003 091 536
- US-A1- 2006 243 676
- US-A1- 2009 220 482
- US-B1- 6 623 472
- YANG S Y ET AL: "Protective effects of IL-1Ra or vIL-10 gene transfer on a murine model of wear debris-induced osteolysis", GENE THERAPY, vol. 11, 2004, pages 483-491, XP002616721,

## Description

### INTRODUCTION

The present technology relates to treatments for osteolysis, including osteolysis related to wear debris resulting from an artificial joint implant in a patient.

Osteolysis involves the dissolution of bone, in particular, the removal or loss of calcium from the bone. Wear debris from artificial joints, such as titanium and polyethylene particulate, can result in an inflammation cascade in the affected joint. For example, human macrophages can respond to various types of wear debris, as described by Rader et al., Cytokine response of human macrophage-like cells after contact with polyethylene and pure titanium particles, J. Arthroplasty: vol. 14, no. 7 (1999); Kaufman et al., Human macrophage response to UHMWPE, TiAlV, CoCr, and alumina particles: analysis of multiple cytokines using protein assays, J. Biomed. Mat. Res. A: DOI 10.1002/jbm.a.; and Matthews et al., Comparison of the response of primary human peripheral blood mononuclear phagocytes from different donors to challenge with model polyethylene particles of known size dose, Biomaterials: vol. 21 (2000). The subsequent inflammation can result in revision of the implant.

Cytokines, such as interleukin-1, play a role in the inflammation cascade and can affect a wide variety of cells and induce many inflammatory reactions, such as fever, production of cytokines, endothelial gene regulation, chemotaxis, leukocyte adherence, and activation of fibroblasts. Methods and treatments to mediate the role of cytokines in the inflammation cascade as it relates to osteolysis would be beneficial. Such treatments may increase the time before revision is necessary or may prevent calcium loss making revision unnecessary.

Yang et al. disclose in Gene Therapy 2004, pages 483 to 491 the results of a study about the protective effect of interleukin-1 receptor antagonist gene transfer on a murine model of wear debris-induced osteolysis.

US 2003/0091536 A1 discloses amethod to reduce the effects of joint disease in a mammal, the method comprising the steps of i) selecting a mammal suspected of having a joint afflicted with a joint disease, ii) administering one or more viral particles to the joint by arthrocentesis; and iii) detecting a reduction in the effects of the joint disease on the treated joint, wherein the viral particles comprise a nucleic acid sequence encoding an equine interleukin-1 receptor antagonist protein.

US 6,623,472 B1 describes a method for preparing at least one therapeutically-effective protein in a syringe, the method comprising the steps of i) placing an inductor, such as immunoglobulin, and a therapeutically-effective protein, such as interleukin 1 receptor antagonist, inside a syringe, ii) filling the syringe with a body fluid and iii) incubating the syringe until additional therapeutically-effective protein is formed in the body fluid.

US 6,096,728 relates to a pharmaceutical composition comprising synergistic amounts of a hyaluronan or a salt thereof, and an interleukin-1 receptor antagonist, wherein the interleukin-1 receptor antagonist comprises all or an interleukin-1 receptor antagonist inhibitory fragment of an amino acid having a specific sequence or a sequence which is at least about 70% homologous to the amino acid sequence.

### SUMMARY

The present invention relates to a composition according to claim 1, and to an implantable device for use in treating osteolysis according to claim 5.

The present technology provides treatments and methods of using solutions rich in interleukin-1 receptor antagonist (IL-lra) and implantable devices for producing IL-1ra for treating osteolysis. The solution and/or device are administered to the site of an artificial joint implant in a human or animal subject where debris from wear of the artificial joint has or is starting to produce inflammation. The present treatments and methods are useful in reducing inflammation resulting from the wear debris. The present treatments and methods can therefore mitigate progression of osteolysis and may also be used to prevent osteolysis.

Interleukin-1 receptor antagonist can be produced by activating adipose tissue, adipocytes, whole blood, platelet rich plasma, and/or white blood cells with polyacrylamide beads or by loading these materials into an implantable device to produce IL-1ra *in vivo.* The adipose tissue, adipocytes, whole blood, platelet rich plasma, and/or white blood cells may be obtained from the subject in order to provide an autologous treatment.

### BRIEF DESCRIPTION OF THE DRAWINGS

The present technology will become more fully understood from the detailed description and the accompanying drawings, wherein:
Figure 1 is a diagrammatic illustration of methods to produce a solution of IL-1ra according to the present technology;
Figure 2 is a diagrammatic illustration of a method for delivering IL-1ra according to an embodiment of the present technology;
Figure 3 is a partial cross-sectional view of a representative device for delivering IL-1ra according to one embodiment of the present technology; and
Figure 4 is an illustration of administration of an implantable device to a knee joint according to an embodiment of the present technology.

It should be noted that the figures set forth herein are intended to exemplify the general characteristics of materials and methods among those of the present technology, for the purpose of the description of certain embodiments. These figures may not precisely reflect the characteristics of any given embodiment, and are not necessarily intended to define or limit specific embodiments within the scope of this technology.

### DETAILED DESCRIPTION

application, or patents issuing therefrom. The following definitions and non-limiting guidelines must be considered in reviewing the description of the technology set forth herein.

The headings (such as "Introduction" and "Summary") and subheadings used herein are intended only for general organization of topics within the present disclosure, and are not intended to limit the disclosure of the technology or any aspect thereof. In particular, subject matter disclosed in the "Introduction" may include novel technology and may not constitute a recitation of prior art. Subject matter disclosed in the "Summary" is not an exhaustive or complete disclosure of the entire scope of the technology or any embodiments thereof. Classification or discussion of a material within a section of this specification as having a particular utility is made for convenience, and no inference should be drawn that the material must necessarily or solely function in accordance with its classification herein when it is used in any given composition.

The citation of references herein does not constitute an admission that those references are prior art or have any relevance to the patentability of the technology disclosed herein.

The description and specific examples, while indicating embodiments of the technology, are intended for purposes of illustration. Moreover, recitation of multiple embodiments having stated features is not intended to exclude other embodiments having additional features, or other embodiments incorporating different combinations of the stated features. Specific examples are provided for illustrative purposes of how to make and use the apparatus and systems of this technology and, unless explicitly stated otherwise, are not intended to be a representation that given embodiments of this technology have, or have not, been made or tested.

As referred to herein, all compositional percentages are by weight of the total composition, unless otherwise specified. As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present technology that do not contain those elements or features.

"A" and "an" as used herein indicate "at least one" of the item is present; a plurality of such items may be present, when possible. "About" when applied to values indicates that the calculation or the measurement allows some slight imprecision in the value (with some approach to exactness in the value; approximately or reasonably close to the value; nearly). If, for some reason, the imprecision provided by "about" is not otherwise understood in the art with this ordinary meaning, then "about" as used herein indicates at least variations that may arise from ordinary methods of measuring or using such parameters. In addition, disclosure of ranges includes disclosure of all distinct values and further divided ranges within the entire range.

The present technology relates to treating osteolysis resulting from wear debris and inflammation at the site of an artificial joint in a patient. Included are treatment methods using interleukin-1 receptor antagonist (IL-1ra), including methods of producing IL-1ra for use in treatments, compositions comprising IL-1ra produced by such methods, and devices for the generation, isolation, and administration of IL-1ra in treatment methods. Methods for treating osteolysis due to wear debris at a site of an artificial joint implant in a patient include administering at or proximate to the wear debris at the site of the artificial joint implant a solution rich in interleukin-1 receptor antagonist.

The present methods and treatments mediate the effects of interleukin-1 and its role in the inflammation cascade. Interleukin-1 (IL-1) includes a family of cytokines that can stimulate lymphocytes and macrophages, activate phagocytes, increase prostaglandin production, contribute to degeneration of bone joints, increase bone marrow cell proliferation, and are involved in many chronic inflammatory conditions. IL-1 can be generated by macrophages, monocytes, and dendritic cells, and can be part of the inflammatory response against infection.

The mode of action of IL-1 can be mediated by interleukin-1 receptor antagonist protein (IL-1ra; also known as "IRAP"). IL-1ra binds to the same receptor on the cell surface as IL-1, and thus prevents IL-1 from sending a signal to that cell. IL-1ra is secreted from white blood cells, including monocytes, macrophages, neutrophils, polymorphonuclear cells (PMNs), and other cells, and can modulate a variety of IL-1 related immune and inflammatory responses, as described by Arend WP, Malyak M, Guthridge CJ, Gabay C (1998) "Interleukin-1 receptor antagonist: role in biology" Annu. Rev. Immunol. 16: 27-55. Production of IL-1ra is stimulated by several substances including adherent immunoglobulin G (IgG), other cytokines, and bacterial or viral components. IL-1ra is an important natural anti-inflammatory protein in arthritis, colitis, and granulomatous pulmonary disease.

IL-1ra can be used in the treatment of rheumatoid arthritis, an autoimmune disease in which IL-1 plays a key role, reducing inflammation and cartilage degradation associated with the disease. For example, Kineret™ (anakinra) is a recombinant, non-glycosylated form of IL-1ra (Amgen Manufacturing, Ltd., Thousand Oaks, California). Various recombinant interleukin-1 inhibitors and methods of treatment are described in U.S. Patent No. 6,599,873, Sommer et al., issued July 29, 2003; U.S. Patent No. 5,075,222, Hannum et al., issued December 24, 1991; and U.S. Application Publication No. 2005/0197293, Mellis et al., published September 8, 2005 In addition, methods for producing IL-1ra from body fluids, including the use of autologous fluids, are described in U.S. Patent No. 6,623,472, Reincke et al., issued September 23, 2003; U.S. Patent No. 6,713,246, Reinecke et al., issued March 30, 2004; and U.S. Patent No. 6,759,188, Reinecke et al., issued July 6, 2004. IL-1ra has also been delivered as part of a composition with hyaluronic acid, as described in U.S. Patent No. 6,096,728, Collins et al., issued August 1, 2000.

The present methods and treatments use solutions rich in IL-1ra that can be produced from the patient's adipose tissue, adipocytes, whole blood, platelet rich plasma, and/or white blood cells, thereby providing an autologous source of IL-1ra. The solution rich in IL-1ra is administered at or proximate to the wear debris at the site of the artificial joint. The IL-1ra acts to block inflammatory cytokines (e.g., IL-1) from binding to the interleukin-1 receptor and therefore slows the progression of inflammation at the site. For example, an autologous IL-1ra rich solution can be produced using a patient's own adipose tissue and/or white blood cells and injected into an affected artificial joint to treat osteolysis and delay or prevent the need for revision of the artificial joint implant.

A solution rich in interleukin-1 receptor antagonist can be produced as described in PCT Application Serial No. PCT/US2009/035541 filed February 27, 2009, U.S. Application Serial No. 12/549,015 filed August 27, 2009, and U.S. Provisional Application Serial No. 61/237,484 filed August 27, 2009.

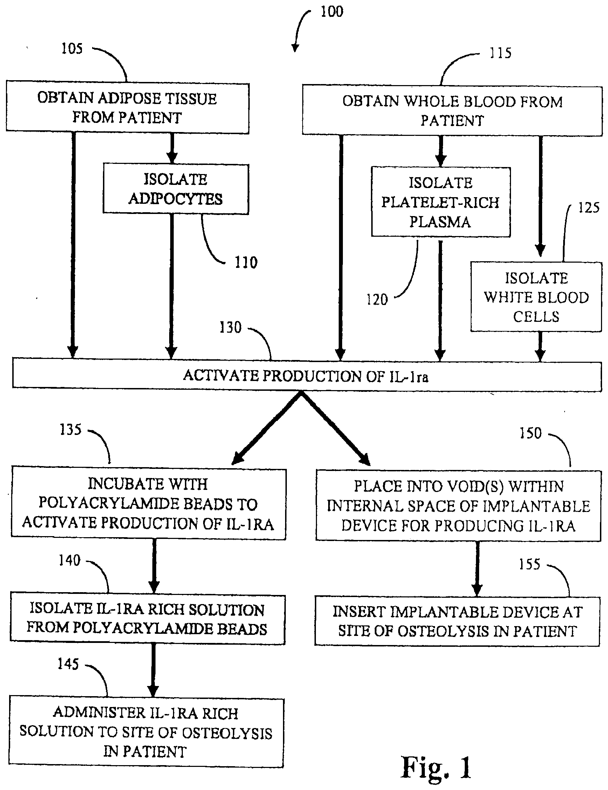

With reference to Figure 1, a treatment method 100 for osteolysis can include the diagrammatically illustrated aspects. Adipose tissue is isolated from a patient at step 105. This adipose tissue may be used directly in step 130, as shown, and the adipose tissue may also be processed to provide adipocytes as shown in step 110. Adipose tissue refers to any fat tissue, either white or brown adipose tissue, which may be derived from subcutaneous, omental/visceral, mammary, gonadal, or other adipose tissue sites. In some embodiments, adipose tissue is derived from human subcutaneous fat isolated by suction assisted lipectomy or liposuction. Adipocytes may be isolated and/or freed from the adipose tissue and/or tissue portions using any suitable method, including methods known in the art such as mechanical and breakdown centrifugation. Adipocytes can also be isolated using enzymatic digestion. For example, adipocytes can be isolated from lipoaspirate, treated by sonication and/or enzymatic digestion, and enriched by centrifugation. Adipocytes isolated from adipose tissue may be washed and pelleted.

Methods for isolating adipose tissue and adipocytes can include the following aspects. Adipose tissue is collected by suction-assisted tumescent liposuction inside a specialized collection container attached to suction hoses and to a liposuction cannula. The collection container can have a gauze-type grid filter that allows the tumescent fluid to pass through and retains the solid adipose tissue. After collecting the adipose tissue, the collection container is removed from the suction device and reattached to a centrifugation device. The filter unit may further contain a filter having approximately a 100 micrometer pore size. Once the collection container containing the adipose tissue is attached to the centrifugation device, the tissue is sonicated. After sonication, the entire apparatus is inserted into a centrifuge bucket and centrifuged at, for example, 300×g for 5 minutes. After centrifugation, the collection container together with the filter unit is detached and can be discarded. The pellet containing the adipocytes can then be resuspended in biocompatible solutions, such as autologous plasma, plasma concentrate and platelet rich plasma.

Adipose tissue may also be treated with digestive enzymes and with chelating agents that weaken the connections between neighboring cells, making it possible to disperse the tissue into a suspension of individual cells, including adipocytes, without appreciable cell breakage. Following disaggregation, the adipocytes may be isolated from the suspension of cells and disaggregated tissue.

Various methods and devices for isolating and/or fractionating adipose tissue and adipocytes include those as described by U.S. Pat. Nos. 7,374,678 and 7,179,391 to Leach et al. and U.S. Pub. Nos. 2009/0014391, 2008/0283474, and 2007/0208321 to Leach et al. A device, such as the GPS™ Platelet Concentrate System (Biomet, Warsaw, IN), may be used to isolate adipocytes. These methods may include obtaining adipocytes by performing lipoaspiration on the patient to obtain adipose tissue, enzymatically digesting the adipose tissue, and separating and/or washing the adipocytes using these devices.

Whole blood is isolated from a patient as shown at step 115 using methods known in the medical arts. The whole blood may be used directly in step 130. In addition, the whole blood may be processed to obtain platelet-rich plasma (PRP), as shown at 120, and may be processed to isolate white blood cells, as shown at 125. For example, PRP contains white blood cells and platelets, which are located in the buffy coat layer following sedimentation of whole blood.

Devices that may be used to isolate platelet-rich plasma at step 120 are also described, for example, in U.S. Patent No. 6,398,972, Blasetti et al., issued June 4, 2002; U.S. Patent No. 6,649,072, Brandt et al., issued November 18, 2003; U.S. Patent No. 6,790,371, Dolocek, issued September 14, 2004; U.S. Patent No. 7,011,852, Sukavaneshvar et al., issued March 14, 2006; U.S. Application Publication No. 2004/0251217, Leach et al., published December 16, 2004; U.S. Application Publication No. 2005/0109716, Leach et al., published May 26, 2005; U.S. Application Publication No. 2005/0196874, Dorian et al., published September 8, 2005; and U.S. Application Publication No. 2006/0175242, Dorian et al., published August 10, 2006.

Other methods may be used to isolate platelet-rich plasma. For example, whole blood can be centrifuged without using a buoy system, whole blood may be centrifuged in multiple stages, continuous-flow centrifugation can be used, and filtration can also be used. In addition, a blood component including platelet-rich plasma can be produced by separating plasma from red blood cells using a slow speed centrifugation step to prevent pelleting of the platelets. In other embodiments, the buffy coat fraction formed from centrifuged blood can be separated from remaining plasma and resuspended to form platelet-rich plasma.

In addition to the GPS® Platelet Concentrate and Separation Systems, a variety of other commercially available devices may be used to isolate platelet-rich plasma at step 120, including the Magellan™ Autologous Platelet Separator System, commercially available from Medtronic, Inc. (Minneapolis, Minnesota, USA); SmartPReP™, commercially available from Harvest Technologies Corporation (Plymouth, Massachusetts, USA); DePuy (Warsaw, Indiana, USA); the AutoloGel™ Process, commercially available from Cytomedix, Inc. (Rockville, Maryland, USA); the GenesisCS System, commercially available from EmCyte Corporation (Fort Myers, Florida, USA); and the PCCS System, commercially available from Biomet 3i, Inc. (Palm Beach Gardens, Florida, USA).

Blood drawn from the patient may be mixed with an anticoagulant. Suitable anticoagulants include heparin, citrate phosphate dextrose (CPD), ethylenediaminetetraacetic acid (EDTA), anticoagulant citrate dextrose solution (ACD), and mixtures thereof. The anticoagulant may be placed in the syringe used for drawing blood from the subject, or may be mixed with the blood after it is drawn.

White blood cells may be prepared, as shown in step 125, using methods known in the art. For example, white blood cells may be prepared from whole blood by lysing red blood cells or by centrifugation of whole blood utilizing a density gradient where the white blood cells sediment to the bottom of a centrifuge tube. An example of density centrifugation includes the Ficoll-Paque™ Plus (GE Healthcare BioSciences, Piscataway, New Jersey, USA). In some cases, a density gradient may be used to further separate mononuclear and polymorphonuclear cells. White blood cells may also be prepared from whole blood using filtration; an example includes the Acelere™ MNC Harvest System (Pall Life Sciences, Ann Arbor, Michigan, USA). White blood cells can also be obtained from bone marrow.

With further reference to Figure 1, in step 130 the production of IL-1ra in the adipose tissue, adipocytes, whole blood, platelet rich plasma, white blood cells, or combinations thereof is activated. These adipose tissue and/or blood materials are activated to produce IL-1ra in two ways: (1) using polyacrylamide beads as shown in steps 135, 140, and 145, and (2) using an implantable device as shown in steps 150 and 155. Just one of these two activation routes may be used or both may be used. In some cases, some of the adipose tissue, adipocytes, whole blood, platelet rich plasma, and/or white blood cells are used in one activation route and some are used in the other activation route. For example, adipocytes may be activated to produce IL-1ra using the implantable device as per steps 150 and 155 and platelet rich plasma is activated to produce IL-1ra using the polyacrylamide beads as per steps 135, 140, and 145. The resultant IL-1ra rich solution and implantable device may then be used in conjunction at a single site of osteolysis or used separately at different sites or proximate sites to treat osteolysis.

Referring again to Figure 1, the first way to produce IL-1ra uses polyacrylamide beads as shown in steps 140, 145, and 150. In this case, the solution rich in IL-1ra is generated by contacting a liquid volume with polyacrylamide beads, wherein the liquid volume comprises a member selected from the group consisting of adipose tissue, adipocytes, whole blood, platelet rich plasma, white blood cells, and combinations thereof. At least a portion of the liquid volume is then separated from the polyacrylamide beads to obtain a solution rich in interleukin-1 receptor antagonist.

Activation to produce IL-1ra using polyacrylamide beads may further include the following aspects. The liquid volume comprising adipose tissue, adipocytes, whole blood, platelet rich plasma, and/or white blood cells may be obtained from the patient that is receiving the treatment for osteolysis so that the patient only receives autologous products. Contacting of the liquid volume with polyacrylamide beads may include incubating the liquid volume with the polyacrylamide beads for a time of from about 30 seconds to about 24 hours. And separating a portion of the liquid volume from the polyacrylamide beads may include centrifuging the liquid volume and polyacrylamide beads to obtain a supernatant comprising the solution rich in interleukin-1 receptor antagonist. Additional aspects and features relating to producing IL-1ra using polyacrylamide beads are described in PCT Application Serial No. PCT/US2009/035541 filed February 27, 2009 and U.S. Application Serial No. 12/549,015 filed August 27, 2009.

As shown at step 135 of Figure 1, the adipose tissue, adipocytes, whole blood, platelet-rich plasma, and/or white blood cells are contacted with polyacrylamide beads. In some embodiments, the adipose tissue, adipocytes, whole blood, platelet rich plasma, and/or white blood cells are incubated with the polyacrylamide beads for a time effective to remove a portion of the liquid in the liquid volume. The incubation may be carried out over a period from about 30 seconds to about 72 hours and may be carried out at a temperature from about 20°C to about 41°C. For example, the incubation may be from about one minute to about 48 hours, from about 5 minutes to about 12 hours, or from about 10 minutes to about 6 hours. In some embodiments, the incubation is conducted at about 37°C. In some embodiments the adipose tissue, adipocytes, whole blood, platelet rich plasma, and/or white blood are not incubated, but are contacted with the polyacrylamide beads for only so long as necessary to perform subsequent processing. The contacting may occur at ambient conditions, e.g., at a temperature of about 20-25°C.

Polyacrylamide beads used in step 135 can be formed by polymerizing acrylamide monomer using controlled and standardized protocols as known in the art to produce relatively uniform beads formed of polyacrylamide gel. In general, polyacrylamide is formed by polymerizing acrylamide with a suitable bifunctional crosslinking agent, most commonly N,N'-methylenebisacrylamide (bisacrylamide). Gel polymerization is usually initiated with ammonium persulfate and the reaction rate is accelerated by the addition of a catalyst, such as N,N,N',N'-tetramethylethylenediamine (TEMED). In various embodiments, polyacrylamide beads comprise 0.5 micromole of carboxyl groups per milliliter of beads, imparting a slight anionic character (negative charge). The beads are also typically resistant to changes in pH, and are stable in many aqueous and organic solutions. By adjusting the total acrylamide concentration, the polyacrylamide gel can be formed in a wide range of pore sizes. Moreover, the polyacrylamide beads can be formed in many sizes and can have relatively uniform size distributions. Bead size may range from several micrometers in diameter to several millimeters in diameter. For example, various types of Bio-Gel™ P polyacrylamide gel beads (Bio-Rad Laboratories, Hercules, California, USA) have particle sizes ranging from less than about 45 µm up to about 180 µm. Polyacrylamide beads are also available from SNF Floerger (Riceboro, Georgia, USA), Pierce Biotechnology, Inc. (Rockford, Illinois, USA), and Polymers, Inc. (Fayetteville, Arkansas, USA).

Once polymerized, polyacrylamide beads can be dried and stored in a powder-like form. The dry beads are insoluble in water but can swell considerably upon being rehydrated. Rehydration returns the polyacrylamide beads to a gel consistency that can be from about two to about three times the dry state size. Thus, dry polyacrylamide beads may be used to absorb a portion of a liquid volume, including solutes smaller than the bead pore size, and can serve to concentrate the IL-1ra produced by the adipocytes and/or adipose tissue. For example, combining dry polyacrylamide beads with the adipocytes and/or adipose tissue in step 130 activates production of IL-1ra and also reduces the total liquid volume as the dry beads rehydrate and swell.

Referring again to Figure 1, following incubation with the polyacrymide beads, an IL-Ira-rich solution is isolated as indicated at step 140. Isolation may be accomplished by drawing off at least a portion of the liquid volume and leaving the beads. In some cases, the beads may be sedimented by centrifugation prior to drawing off the IL-Ira-rich solution. Isolation may also be performed by filtration, where the polyacrylamide beads are retained by a filter and the IL-1ra-rich solution passes through the filter using centrifugal force or by using vacuum, for example. If the incubation with polyacrylamide beads at step 135 utilizes dry polyacrylamide beads, the liquid volume may be reduced as the beads swell upon rehydration, thereby concentrating the resulting IL-1ra-rich solution. To maintain the increased concentration, care should be taken in the isolation step 140 so as to avoid compressing the beads or drawing liquid out from the swollen beads. For example, high centrifugal force or high vacuum may collapse the beads and/or draw liquid out of the internal volume of the beads.

Without limiting the mechanism, utility or function of the present technology, the polyacrylamide beads serve as an activator of IL-1ra production by adipose tissue, adipocytes, whole blood, platelet rich plasma, and/or white blood cells. Therefore, in the case of dry polyacrylamide beads, not only is liquid being absorbed from the liquid volume comprising adipose tissue, adipocytes, whole blood, platelet rich plasma, and/or white blood cells, thereby concentrating the IL-1ra formed, but the beads further serve as a surface to stimulate IL-1ra production. It appears that the increase in the amount of IL-1ra is not due to simply increasing the concentration by reducing the volume of the sample, but is due to activation of the adipose tissue, adipocytes, whole blood, platelet rich plasma, and/or white blood cells by the polyacrylamide beads to increase production and/or release of IL-1ra. There also appears to be a correlation between the amount of IL-1ra produced and the concentration of white blood cells, where adipose tissue can include white blood cells. Thus, the present technology uses adipose tissue and disaggregated adipose tissue to obtain adipocytes, where white blood cells can be present in both the adipose tissue and the adipocytes obtained from adipose tissue.

Activation of the production of IL-1ra may include the use of a device such as the Plasmax™ Plus Plasma Concentrator, from Biomet Biologics, LLC (Warsaw, Indiana, USA) and may include those devices and methods of use as described in U.S. Application Publication 2006/0175268, Dorian et al., published August 10, 2006; and U.S. Application Publication 2006/0243676, Swift et al., published November 2, 2006.

Referring again to Figure 1, in step 145 the IL-1ra-rich solution is administered to a human or animal subject (i.e., patient). The patient receiving the IL-1ra-rich solution may be the same patient from which the adipose tissue, adipocytes, whole blood, platelet rich plasma, and/or white blood cells in steps 105 and/or 115 are derived. In this case, the method provides an autologous preparation of IL-1ra. Administration may be performed using various means, such as by injection of the IL-1ra-rich solution using a syringe, surgical application, or application concomitant with another surgical procedure. It should be understood, however, that step 145 may comprise any biomedically acceptable process or procedure by which the IL-1ra-rich solution is implanted, injected, or otherwise administered in or in proximity to a site in order to mediate effects related to stimulation of the interleukin-1 receptor, such as inflammation and inflammation due to osteoarthritis. For example, the IL-1ra rich solution is used for treating osteolysis at the site of an artificial joint implant. Injection may be located at or into the synovial space of the joint, or otherwise at or near the joint.

The various preparations of IL-1ra-rich solutions produced by the present technology may be sterilized by including a sterile filter to process the final isolated IL-1ra product. Similarly, an antibiotic may be included in the polyacrylamide beads during incubation or added at one or more of the various steps in the methods and treatments described herein.

Solutions rich in IL-1ra generated in step 140 of Figure 1 can be characterized as having increased concentrations of IL-1ra relative to the concentration of IL-1ra typically found in whole blood. For example, the present methods and compositions can include about 34,000 pg/mL to about 108,000 pg/mL of IL-1ra, whereas whole blood can include about 200 pg/mL to about 800 pg/mL. It is understood, however, the concentrations present in any given solution may vary depending on the initial levels of components present in the adipose tissue, adipocytes, and/or source of white blood cells used in the present methods, and that increases in concentration are relative to those initial levels. According to the present invention, IL-Ira is present in the present solutions at concentrations of at least about 10,000 pg/ml, preferably at least about 25,000 pg/ml, or at least about 30,000 pg/ml and can be up to 108,000 pg/mL.

In some embodiments, the present methods include administering fibrinogen, thrombin, and calcium to the treatment site in addition to administering the solution rich in interleukin-1 receptor antagonist. For example, methods can include co-administering (i) a first solution comprising the interleukin-1 receptor antagonist and fibrinogen, and (ii) a second solution comprising thrombin and calcium. Thrombin used in the present methods may be made by a process that includes loading whole blood or plasma and a calcium solution into a blood isolation device. The whole blood or plasma is heated for at least about 20 minutes, at a temperature of at least about 20°C. Thrombin is isolated by centrifuging the heated whole blood or plasma. The whole blood or plasma may be obtained from the patient. The solution rich in interleukin-1 receptor antagonist and the clotting fraction are then administered to the site of the inflammation in the patient.

Referring again to Figure 1, the second way to activate production of IL-1ra uses an implantable device as shown in steps 150 and 155. In this case, the implantable device is loaded with a liquid volume in step 150, where the liquid volume comprises a member of the group consisting of adipose tissue, adipocytes, whole blood, platelet rich plasma, white blood cells, and combinations thereof. The implantable device is then inserted into the patient at or proximate to the treatment site, as shown at 155. The implantable device produces interleukin-1 receptor antagonist *in vivo* to treat osteolysis due to wear debris at the site of the artificial joint implant in the patient.

The implantable device comprises an enclosed or substantially enclosed body defining an internal space, wherein at least a portion of the body comprises a first bioresorbable material. The internal space includes a second bioresorbable material having an activation surface and the internal space includes one or more voids. The liquid volume of adipose tissue, adipocytes, whole blood, platelet rich plasma, and/or white blood cells is located within the one or more voids.

Activation to produce IL-1ra using the implantable device may further include the following aspects. The liquid volume comprising adipose tissue, adipocytes, whole blood, platelet rich plasma, white blood cells, and combinations thereof can be obtained from the patient. The activation surface of the second bioresorbable material can comprise immunoglobulin G. Additional aspects and features relating to producing IL-1ra using such implantable devices are described in U.S. Provisional Application Serial No. 61/237,484 filed August 27, 2009.

With reference to Figure 1, the IL-1ra rich solution obtained at step 140 and/or the loaded implantable device at 150 are then administered 145 or inserted 155 to treat osteolysis due to wear debris at the site of an artificial implant. The IL-Ira-rich solutions and/or implantable device can mediate the effects of IL-1 and attenuate signaling via the interleukin-1 receptor. Blocking the biological activity of naturally occurring IL-1 can treat inflammation and cartilage degradation associated with osteolysis by competitively inhibiting the binding of IL-1 to the interleukin-1 type receptor, which is expressed in many tissues and organs. For example, bone resorption and tissue damage such as cartilage degradation as a result of loss of proteoglycans due to IL-1 may be treated by administration of the IL-Ira-rich solution and/or implantable device. In some patients, endogenous IL-1ra may not be found in effective concentrations in synovium and synovial fluid to counteract IL-1 concentrations, and hence the present IL-1ra-rich solution and/or implantable device may be administered to treat these conditions and these sites. Dosing, administration, and frequency of treatment may be modified based on established medical practices to achieve effective treatment.

In some embodiments, an IL-1ra-rich solution and an implantable device are co-administered to a site of osteolysis. In this way, the IL-1ra-rich solution can provide a more immediate effect in blocking IL-1 binding to the interleukin-1 receptor to mediate the inflammation cascade and role of IL-1 in osteolysis. The implantable device, on the other hand, can provide a reservoir for the production of IL-1ra that may be more slowly released over time. In particular, the bioresorbable materials of the implantable device may erode or degrade over the course of several days or a week or more while providing a release of IL-1ra during this time. For example, an injection of IL-1ra rich solution can provide an immediate attenuation of the effect of IL-1 in osteolysis and the implantable device can provide a sustained attenuation of the effect of IL-1 in osteolysis.

The present technology provides improved treatments by using autologous materials to activate production of autologous IL-1ra to treat a patient. Hence immunological issues that may arise when using non-autologous material or recombinant material are reduced and/or substantially eliminated. Moreover, since the IL-1ra is produced by the patient's cells, natural post-translational modifications, such as glycosylation, are already present. This is not the case with most recombinant proteins since they are produced in prokaryotic hosts.

In cases where IL-1ra-rich solutions are used, administration of such solutions can include fibrinogen where the fibrinogen is activated to form a fibrin matrix that protects and retains the IL-1ra at a treatment site. The fibrin matrix can be formed *in situ* upon delivery of the IL-1ra at or proximate to the site of osteolysis.

Fibrinogen can be cross-linked into a three-dimensional matrix by activation with a clotting agent and calcium. Suitable clotting agents include thrombin (e.g., bovine, recombinant human, pooled human, or autologous), autologous clotting protein, and polyethylene glycol. Calcium may be in the form of a calcium salt, such as calcium chloride.

In some embodiments, the clotting agent comprises an autologous clotting protein, as a clotting fraction or composition derived from blood obtained from the patient to be treated. A suitable clotting fraction can be obtained by a process of: loading whole blood or plasma with a calcium solution (e.g., calcium chloride in ethanol) into a blood isolation device; heating the whole blood or plasma for at least about 20 minutes, at a temperature of at least about 20°C; and isolating the clotting fraction. The isolating may be performed by centrifuging the heated whole blood or plasma. A suitable isolation device is available as the Clotalyst™ Autologous Thrombin Collection System from Biomet Biologics LLC, Warsaw, Indiana, USA.

Administration of the IL-1ra can therefore further include fibrinogen, thrombin, and calcium to form a fibrin matrix at the treatment site. Exogenous fibrinogen may be added to a solution of IL-1ra, for example such as bovine thrombin, preferably at about 1,000 U/mL. Or, the IL-1ra solution may already have an adequate amount of endogenous fibrinogen. In the case where the solution of IL-1ra and/or fibrinogen or preparation thereof includes an anticoagulant, such as ACD-A (anticoagulant citrate dextrose solution), the addition of calcium (with thrombin) to activate the fibrinogen should exceed the effective amount of any chelator (e.g., EDTA) in the anticoagulant.

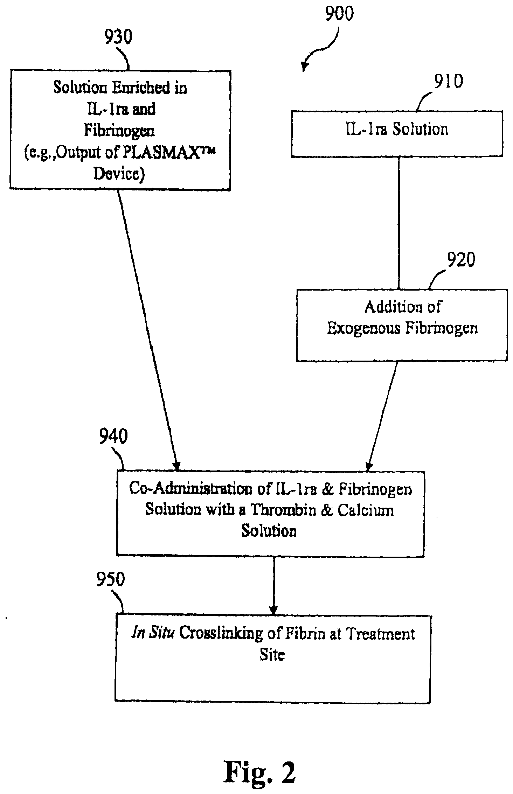

Referring to Figure 2, a diagrammatic illustration for delivering IL-1ra 900 is shown. At step 910, a solution of IL-1ra is provided. The IL-1ra solution may be prepared using the methods and treatments as described. Exogenous fibrinogen is added to the IL-1ra solution in step 920. The exogenous fibrinogen may be prepared from a different source than the IL-1ra solution, such as a different patient, or may be bovine in origin. Or, the exogenous fibrinogen may be prepared from different starting material than the IL-1ra solution, but still from the same source or patient. For example, the IL-1ra solution and the exogenous fibrinogen may be prepared from different blood samples taken from the same patient. Alternatively, as shown in step 930, a solution that is enriched in both IL-1ra and fibrinogen is prepared, for example, by using polyacrylamide beads and the Plasmax™ device, as described. A solution of thrombin and calcium is provided in step 940 and is co-administered with the solution of IL-1ra to a treatment site. Thereafter, as shown in step 950, the fibrin in the combined solutions cross-links *in situ,* forming a matrix at the treatment site that serves to protect, retain, and slow release of the IL-1ra.

Delivery of IL-1ra may include co-administering a first solution of IL-1ra and fibrinogen and a second solution of thrombin and calcium to a subject. In such embodiments, the first solution and second solution are kept separate until administered so that the fibrinogen does not form a fibrin matrix until after the solutions are mixed and injected into a treatment site. The solutions may be mixed just before delivery to the treatment site or may be mixed at the treatment site.

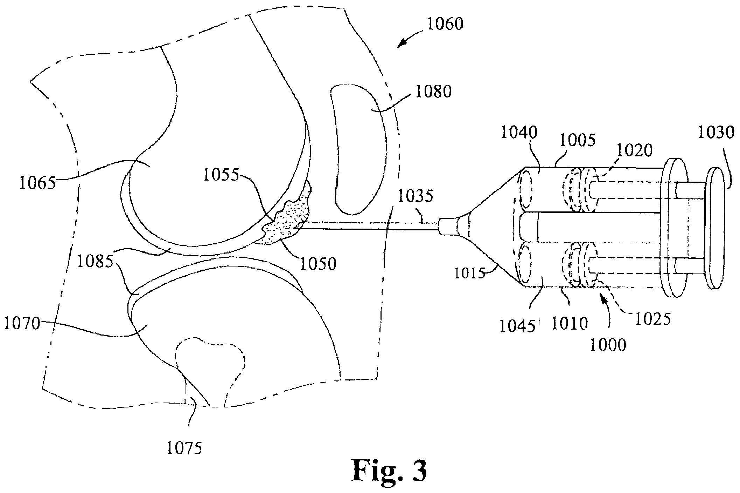

Referring to Figure 3, a dual syringe device 1000 may be employed in a medically appropriate procedure. The dual syringe device 1000 includes a first barrel 1005 and a second barrel 1010, both of which are connected to a mixing chamber 1015. A first plunger 1020 is inserted into the first barrel 1005 and a second plunger 1025 is inserted into the second barrel 1010. The first plunger 1020 and the second plunger 1025 are connected by a member 1030. The mixing chamber 1015 connects to a cannula 1035. The dual syringe device 1000 contains a first solution 1040 of IL-1ra and fibrinogen in the first barrel 1005, and a second solution 1045 of thrombin and calcium in the second barrel 1010. During co-administration, member 1030 is pushed toward the mixing chamber 1015 such that the contents of both the first barrel 1005 and the second barrel 1010 are pushed into the mixing chamber 1015. The mixed first solution 1040 and second solution 1045 travel through the cannula 1035 and form a fibrin-matrix 1050 at the treatment site 1055 within a patient's joint 1060.

In the embodiment shown in Figure 3, the patient's joint 1060 includes an artificial joint implant as part of the knee joint. The artificial joint implant may comprise one or more portions of the joint, including a femur 1065, a tibia 1070, a fibula 1075, a patella 1080, and cartilage 1085. For example, in one embodiment of an artificial knee joint, all or some of the cartilage 1085 may be missing and an artificial portion of the femur 1065 may be contacting an artificial portion of the tibia 1070. Friction between the portions of the artificial results in the formation of wear debris, such as polyethylene or titanium particles. These particles can provoke the inflammation cascade and induce osteolysis at the artificial joint implant. Figure 3 illustrates a knee joint, however, it should be understood that the treatment site 1055 may be any artificial joint, including shoulders, elbows, wrists, ankles, hips, and the spinal column. In addition, the present treatments and methods may be applied to treat inflammation at artificial implants within other tissues, such as muscle and tendon.

In some embodiments, the dual syringe device 1000 is used to pierce soft tissue of the patient's joint 1060 to administer the mixed first solution 1040 and second solution 1045. For example, the cannula 1035 may be a hollow needle such as a hypodermic needle. Alternatively, an incision may be made in the patient's joint 1060 to allow entry of the cannula 1035 so that the dual syringe device 800 may enter the treatment site 1055.

In some embodiments, which are not shown, the dual syringe device 1000 does not have a mixing chamber 1015 and instead includes two cannulas 1035, one leading from each barrel to the treatment site 1055. In this case, the first solution 1040 and second solution 1045 travel through the separate cannulas 1035 and mix together at the treatment site 1055 to form a fibrin-matrix 1050. In some embodiments, two separate single-barreled syringe devices are employed in place of a dual syringe device.

The fibrin matrix formed in the present delivery methods can reside at the treatment site without increasing inflammation. The IL-1ra within the fibrin matrix is protected from enzymatic degradation and may bind to the fibrin matrix so that is it slowly released from the matrix over time. The methods consequently can provide sustained delivery of IL-1ra as compared to injection of IL-1ra without the fibrin-matrix carrier.

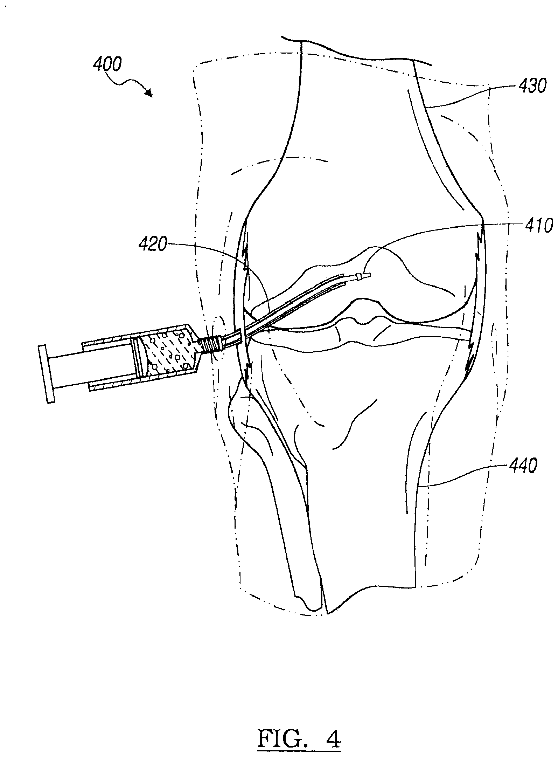

Referring now to Figure 4, an embodiment of a treatment 400 using an embodiment of the present implantable device 410 is shown. The implantable device 410 is inserted at the site of osteolysis using a cannulated device 420. A movable element (not shown) is disposed within a portion of the cannulated device 420 and the implantable device 410 is originally disposed within a portion of the cannula device 420. The movable element is operable to expel the implantable device 410 from the cannulated device 420 into position at or near the artificial joint, which in Figure 4 is depicted as a knee joint. The implantable device 410 loaded with adipose tissue, adipocytes, whole blood, platelet-rich plasma, and/or white blood cells produces interleukin-1 receptor antagonist *in vivo* at the implantation site. As shown in Figure 4, the implantation site is proximate to the end of the patient's femur near the knee joint.

The present technology can include aspects of U.S. Provisional Application No. 61/031,803 filed February 27, 2008, U.S. Provisional Application No. 61/116,940 filed November 21, 2008, and U.S. Provisional Application No. 61/155,048 filed February 24, 2009 and includes aspects of PCT/US2009/035541 filed February 27, 2009.

The following specific examples are provided for illustrative purposes of how to make and use the compositions and methods of this technology and, unless explicitly stated otherwise, are not intended to be a representation that given embodiments of this technology have, or have not, been made or tested.

### EXAMPLE 1

Adipocytes are prepared as follows. Adipose tissue is harvested by liposuction from a patient, minced into small pieces (about 1 cm³), and digested in 2 mg/mL type I collagenase (Worthington Biochemical Corp., Lakewood, N.J.) under intermittent mechanical agitation in a water bath at 37°C for 180 minutes. Digestion is neutralized by the addition of whole blood obtained from the patient. The cell suspension is centrifuged (300×g for 7 minutes at 25°C) followed by removal of the supernatant from the cell pellet. The pellet is then resuspended in platelet rich plasma to provide a liquid volume comprising adipocytes and platelet rich plasma.

The adipocytes and platelet rich plasma are combined with polyacrylamide beads to activate production of IL-1ra. After incubating for about 4 hours, the mixture is centrifuged and the supernatant is isolated to provide an IL-1ra rich solution. The IL-1ra rich solution is administered to an artificial knee joint in the patient by injection using a syringe. The treatment reduces or eliminates progression of osteolysis at the artificial knee joint.

### EXAMPLE 2

A method for treating osteolysis due to wear debris at a site of an artificial joint implant in a patient includes using an implantable device to produce interleukin-1 receptor antagonist *in vivo.* The implantable device includes an enclosed tubular body including a lumen where the tubular body comprises a first bioresorbable material. A second bioresorbable material is within the lumen of the tubular body. The second bioresorbable material comprises a plurality of gelatin beads from about 10 microns to about 20 microns in diameter. One end of the tubular body comprises a self-healing surface that can be pierced with one or more needles and adipose tissue, adipocytes, whole blood, PRP, and/or white blood cells are loaded into the lumen of the device. The self-healing surface is operable to substantially seal the puncture hole once loading is complete and the needle(s) withdrawn. In this way, the beads of the second bioresorbable material and the loaded adipose tissue, adipocytes, whole blood, PRP, and/or white blood cells do not leak out of the device.

The implantable device is then inserted proximate to an artificial hip in a patient. The implantable device produces IL-1ra that is slowly released over the course of a week as the bioresorbable materials of the implantable device degrade. The sustained release of IL-1ra halts the progression of osteolysis and attenuates inflammation at the artificial hip joint.

The examples and other embodiments described herein are exemplary and not intended to be limiting in describing the full scope of compositions and methods of this technology. Equivalent changes, modifications and variations of specific embodiments, materials, compositions and methods may be made within the scope of the present claims.

## Claims

1. A composition for use in the treatment of osteolysis due to wear debris at a site of an artificial joint implant in a patient, the composition comprising a solution rich in interleukin-1 receptor antagonist, wherein the interleukin-1 receptor antagonist is autologous to the patient, wherein the solution rich in interleukin-1 receptor antagonist is made by:
a. contacting a liquid volume with polyacrylamide beads, wherein the liquid volume comprises a member selected from the group consisting of adipose tissue, adipocytes, whole blood, platelet rich plasma, white blood cells, and combinations thereof; and
b. separating at least a portion of the liquid volume from the polyacrylamide beads to obtain a solution rich in interleukin-1 receptor antagonist,
wherein the solution comprises interleukin-1 receptor antagonist at a concentration of at least 10,000 pg/mL, preferably from 30,000 pg/mL and can be up to 108,000 pg/mL.

2. A composition for use according to claim 1, wherein the treatment further comprises administering fibrinogen, thrombin, and calcium with the solution rich in interleukin-1 receptor antagonist.

3. A composition for use according to claim 1, wherein the liquid volume is obtained from the patient.

4. A composition for use according to claim 1, wherein the separating comprises centrifuging the liquid volume and polyacrylamide beads to obtain a supernatant comprising the solution rich in interleukin-1 receptor antagonist.

5. An implantable device for producing interleukin-1 receptor antagonist *in vivo* for use in treating osteolysis due to wear debris at a site of an artificial joint implant in a patient, wherein the implantable device is implanted by inserting it at the site of osteolysis using a cannulated device, wherein the interleukin-1 receptor antagonist is autologous to the patient, the implantable device comprising:
an enclosed body defining an internal space, wherein a part or all of the body comprises a first bioresorbable material; and
a second bioresorbable material within the internal space, wherein the second bioresorbable material includes an activation surface comprising immunoglobulin G;
one or more voids within the internal space; and
a liquid volume within the one or more voids, wherein the liquid volume comprises a member selected from the group consisting of adipose tissue, adipocytes, whole blood, platelet rich plasma, white blood cells, and combinations thereof,
wherein the activation surface activates the adipose tissue, adipocytes, whole blood, platelet rich plasma, white blood cells, and combinations thereof to generate the interleukin-1 receptor antagonist.

6. The implantable device for use according to Claim 5, wherein the liquid volume is obtained from the patient.

## Patentansprüche

1. Zusammensetzung zur Verwendung in der Behandlung von Osteolyse aufgrund von Abrieb an einer Stelle eines künstlichen Gelenkimplantats in einem Patienten, wobei die Zusammensetzung eine Lösung umfasst, die reich an Interleukin-1-Rezeptor-Antagonist ist, wobei der Interleukin-1-Rezeptor-Antagonist dem Patienten autolog ist, wobei die Lösung, die reich an Interleukin-1-Rezeptor-Antagonist ist, hergestellt ist durch:
a. in Kontakt Bringen eines Flüssigkeitsvolumens mit Polyacrylamid-Kügelchen, wobei das Flüssigkeitsvolumen einen Bestandteil umfasst, welcher ausgewählt ist aus der Gruppe bestehend aus Fettgewebe, Fettzellen, Vollblut, plättchenreichem Plasma, weißen Blutzellen und Kombinationen derselben; und
b. Trennen zumindest eines Teils des Flüssigkeitsvolumens von den Polyacrylamid-Kügelchen, um eine Lösung zu erhalten, die reich an Interleukin-1-Rezeptor-Antagonist ist,
wobei die Lösung Interleukin-1-Rezeptor-Antagonist in einer Konzentration von mindestens 10000 pg/mL, bevorzugt ab 30000 pg/mL umfasst, die bis zu 108000 pg/mL betragen kann.

2. Zusammensetzung zur Verwendung nach Anspruch 1, wobei die Behandlung zudem umfasst, dass mit der Lösung, die reich an Interleukin-1-Rezeptor-Antagonist ist, Fibrinogen, Thrombin und Calcium verabreicht werden.

3. Zusammensetzung zur Verwendung nach Anspruch 1, wobei das Flüssigkeitsvolumen vom Patienten erhalten wird.

4. Zusammensetzung zur Verwendung nach Anspruch 1, wobei das Trennen umfasst, dass das Flüssigkeitsvolumen und die Polyacrylamid-Kügelchen zentrifugiert werden, um einen Überstand zu erhalten, welcher die Lösung umfasst, die reich an Interleukin-1-Rezeptor-Antagonist ist.

5. Implantierbare Vorrichtung zur Erzeugung von Interleukin-1-Rezeptor-Antagonist *in vivo,* zur Verwendung in der Behandlung von Osteolyse aufgrund von Abrieb an einer Stelle eines künstlichen Gelenkimplantats in einem Patienten, wobei die implantierbare Vorrichtung durch Einführen an der Stelle der Osteolyse unter Verwendung einer kanülierten Vorrichtung implantiert wird, wobei der Interleukin-1-Rezeptor-Antagonist dem Patienten autolog ist, wobei die implantierbare Vorrichtung umfasst:
einen umschlossenen Körper, der einen Innenraum definiert, wobei ein Teil oder das Ganze des Körpers ein erstes bioresorbierbares Material umfasst; und
ein zweites bioresorbierbares Material innerhalb des Innenraums, wobei das zweite bioresorbierbare Material eine Aktivierungsoberfläche enthält, die Immunglobulin G umfasst;
einen oder mehrere Hohlräume innerhalb des Innenraums; und
ein Flüssigkeitsvolumen innerhalb des einen oder der mehreren Hohlräume, wobei das Flüssigkeitsvolumen einen Bestandteil umfasst, welcher ausgewählt ist aus der Gruppe bestehend aus Fettgewebe, Fettzellen, Vollblut, plättchenreichem Plasma, weißen Blutzellen und Kombinationen derselben,
wobei die Aktivierungsoberfläche das Fettgewebe, die Fettzellen, das Vollblut, das plättchenreiche Plasma, die weißen Blutzellen und die Kombinationen derselben aktiviert, um den Interleukin-1-Rezeptor-Antagonisten zu erzeugen.

6. Implantierbare Vorrichtung zur Verwendung nach Anspruch 5, wobei das Flüssigkeitsvolumen vom Patienten erhalten wird.

## Revendications

1. Composition destinée à être utilisée dans le traitement de l'ostéolyse due à des débris d'usure à un site d'un implant d'articulation artificielle chez un patient, la composition comprenant une solution riche en antagoniste du récepteur de l'interleukine-1, dans laquelle l'antagoniste de récepteur d'inter-leukine-1 est autologue au patient, dans laquelle la solution riche en antagoniste de récepteur d'interleukine-1 est constituée par :
a. la mise en contact d'un volume de liquide avec des billes de polyacrylamide, dans laquelle le volume de liquide comprend un élément choisi dans le groupe constitué par le tissu adipeux, les adipocytes, le sang entier, le plasma riche en plaquettes, les globules blancs du sang et leurs combinaisons ; et
b. la séparation d'au moins une partie de volume de liquide des billes de polyacrylamide pour obtenir une solution riche en antagoniste du récepteur de l'interleukine-1, dans laquelle la solution comprend l'antagoniste de l'interleukine-1 à une concentration d'au moins 10000 pg/mL, de préférence de 30000 pg/mL et peut être jusqu'à 108000 pg/mL.

2. Composition destinée à être utilisé selon la revendication 1, dans laquelle le traitement comprend en outre l'administration de fibrinogène, de thrombine et de calcium avec la solution riche en antagoniste du récepteur de l'inter-leukine-1.

3. Composition destinée à être utilisée selon la revendication 1, dans laquelle le volume de liquide est obtenu du patient.

4. Composition destinée à être utilisée selon la revendication 1, dans laquelle la séparation comprend la centrifugation du volume de liquide et des billes de polyacrylamide pour obtenir un surnageant comprenant la solution riche en antagoniste du récepteur de l'interleukine-1.

5. Dispositif implantable pour produire l'antagoniste de récepteur d'interleu-kine-1 *in vivo* destiné à être utilisé dans le traitement de l'ostéolyse due aux débris d'usure à un site d'un implant d'articulation artificielle chez un patient, ledit dispositif implantable étant implanté en l'insérant au site d'ostéolyse en utilisant un dispositif canulé, dans lequel l'antagoniste du récepteur de l'interleukine-1 est autologue au patient, le dispositif implantable comprenant :
un corps enfermé définissant un espace interne, dans lequel une partie ou l'ensemble du corps comprend un premier matériau biorésorbable ; et
un second matériau biorésorbable dans l'espace interne, dans lequel le second matériau biorésorbable comprend une surface d'activation comprenant une immunoglobuline G ;
un ou plusieurs vides dans l'espace interne ; et
un volume de liquide avec le ou les vides, dans lequel le volume de liquide comprend un élément choisi dans le groupe constitué par le tissu adipeux, les adipocytes, le sang entier, le plasma riche en plaquettes, les globules blancs du sang et leurs combinaisons,
dans lequel la surface d'activation active le tissu adipeux, les adipocytes, le sang entier, le plasma riche en plaquettes, les globules blancs du sang et leurs combinaisons pour produire l'antagoniste du récepteur de l'interleu-kine-1.

6. Dispositif implantable destiné à être utilisé selon la revendication 5, dans lequel le volume de liquide est obtenu du patient.
